# DEMANDE DE BREVET EUROPEEN

(11) **EP 0 899 257 A1**
(43) Date de publication de la demande: **03.03.1999**
(21) Numéro de dépôt: 98402026.3
(22) Date de dépôt: 10.08.1998
(51) Int. Cl.: C07C 319/18, C07C 323/52

(54) **Synthèse d'acides carboxyalkylthiosucciniques**

(30) Priorité: 27.08.1997 FR 9710707
(71) Demandeur: ELF ATOCHEM S.A., 92800 Puteaux, Hauts-de-Seine (FR)
(72) Inventeur: Labat, Yves, 33110 Le Bouscat (FR); Muller, Jean-Pierre, 64360 Monein (FR)
(74) Mandataire: Leboulenger, Jean

(57) **Abrégé**

Pour préparer les acides carboxyalkylthiosucciniques de formule : 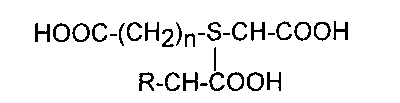 dans laquelle n = 1 à 3 et R = H ou CH₃, on fait réagir en milieu aqueux, sans catalyseur, un mercapto-ester de formule : HS-(CH₂)ₙ-COOR' dans laquelle R' est un radical alkyle en C₁ à C₄, avec l'acide maléique, l'acide citraconique ou leur anhydride.

L'invention vise plus particulièrement la préparation de l'acide carboxyéthylthiosuccinique (CETSA).

## Description

La présente invention qui concerne le domaine des acides polycarboxyliques a pour objet la synthèse d'acides carboxyalkylthiosucciniques et plus particulièrement celle de l'acide carboxyéthylthiosuccinique (ci-après désigné par l'abréviation CETSA).

Ce composé connu de formule : 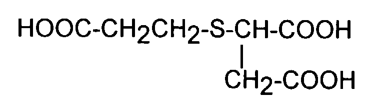 est notamment utile comme inhibiteur de corrosion (demande de brevet JP 08-277481), comme additif de façonnage de polymères (demande de brevet JP 08-259732) ou encore comme constituant de compositions détergentes (demande de brevet JP 08-283783).

Selon la demande de brevet JP 08-311055, le CETSA est préparé par réaction de l'acide mercaptopropionique avec l'anhydride maléique, au sein d'un solvant polaire tel que la méthyléthylcétone et en présence d'un catalyseur constitué par une résine échangeuse d'ions. L'acide mercaptopropionique est généralement obtenu par hydrolyse acide des mercaptopropionates d'alkyle et plus particulièrement celle du mercaptopropionate de méthyle (ci-après MMP).

Il a maintenant été trouvé que le CETSA peut être obtenu directement et sans catalyseur par réaction du MMP avec l'acide maléique ou son anhydride en milieu aqueux. De manière surprenante, l'addition d'un catalyseur acide tel que l'acide sulfurique ou l'acide p-toluènesulfonique n'est pas nécessaire pour la réaction d'addition de la fonction SH du MMP sur la double liaison, ni surtout pour l'hydrolyse de l'ester intermédiaire en CETSA:

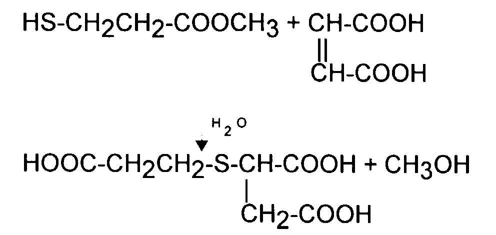

En remplaçant l'acide maléique par l'acide citraconique ou son anhydride, on peut obtenir de la même façon l'acide 2-carboxyéthylthio-3-méthylsuccinique. On peut également remplacer le MMP par d'autres mercapto-esters tels que, par exemple, le thioglycolate de méthyle, le thioglycolate d'éthyle ou le mercaptopropionate d'éthyle.

L'invention a donc pour objet un procédé de préparation d'acides carboxyalkylthiosucciniques de formule générale : 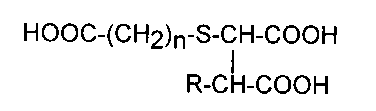 dans laquelle n est un nombre entier allant de 1 à 3 et R désigne un atome d'hydrogène ou un radical méthyle caractérisé en ce qu'il consiste essentiellement à faire réagir, en milieu aqueux et en l'absence de catalyseur, un mercapto-ester de formule générale :

HS-(CH₂)ₙ-COOR'

dans laquelle n a la même signification que ci-dessus et R' représente un radical alkyle contenant de 1 à 4 atomes de carbone, avec l'acide maléique, l'acide citraconique ou leur anhydride.

La réaction peut être réalisée à une température comprise entre 60 et 140°C, mais est avantageusement effectuée entre 80 et 120°C et, encore mieux, au reflux. Selon la température de réaction choisie et la composition du mélange eau-alcool à évacuer, la pression peut aller de 1 à 4 bars ; elle est de préférence comprise entre 1 et 2 bars et, mieux encore, égale à la pression atmosphérique.

Les réactifs (mercapto-ester et acide ou anhydride maléique ou citraconique) sont de préférence utilisés en quantités sensiblement équimolaires, mais on ne sortirait pas du cadre de la présente invention en les utilisant dans un rapport molaire mercapto-ester/acide ou anhydride maléique ou citraconique allant de 0,8 à 1,4, en particulier entre 1 et 1,2.

La réaction est avantageusement effectuée en discontinu (batch) mais elle peut aussi être menée en continu en réacteurs agités en cascade. Que l'on travaille en batch ou en continu, il est indispensable de distiller l'alcool R'OH libéré. La teneur en réactifs et/ou acide carboxyalkylthiosuccinique du milieu réactionnel peut varier dans de larges proportions pendant la synthèse, compte-tenu de l'évacuation permanente d'un mélange eau-alcool ; un large excès d'eau favorise le départ de l'alcool, mais peut freiner l'achèvement de la réaction. Pour assurer un reflux régulier et la meilleure élimination possible de l'alcool, il est avantageux en cours de synthèse d'ajouter périodiquement de l'eau. Il est également recommandé de maintenir dans le milieu réactionnel une concentration de mercapto-ester comprise entre 2 et 5 moles/litre de solution.

L'acide carboxyalkylthiosuccinique peut être isolé du mélange réactionnel par précipitation lors du retour à la température ambiante; il peut être purifié par recristallisation dans l'eau.

L'exemple suivant illustre l'invention sans la limiter.

### EXEMPLE

Dans un réacteur agité et thermostaté, on a introduit 82 g (0,83 mole) d'anhydride maléique et 250 g d'eau, puis en 30 minutes on a porté le mélange au reflux. On a alors introduit 100 g (0,83 mole) de MMP et chauffé le mélange à reflux pendant 2 heures. On a distillé en continu le méthanol libéré et maintenu constant le volume réactionnel par addition d'eau (620 g). Après 8 heures au reflux à 100°C, on a refroidi et obtenu un solide blanc précipité qui, par filtration, a fourni 240 g d'un produit contenant 121 g (0,545 mole) de CETSA, ainsi que 0,5 g de MMP, 11 g de thioesters et 108 g d'eau. Le filtrat (131 g) qui contenait 52 g (0,23 mole de CETSA) peut être recyclé dans une nouvelle opération.

Après séchage, on a obtenu un produit brut fondant à 152°C avec une pureté, mesurée par RMN de 93 %.

## Revendications

1. Procédé de préparation d'un acide carboxyalkylthiosuccinique de formule générale : 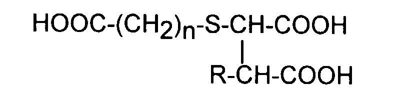 dans laquelle n est un nombre entier allant de 1 à 3 et R est un atome d'hydrogène ou un radical méthyle caractérisé en ce qu'il consiste essentiellement à faire réagir en milieu aqueux un mercapto- ester de formule générale :
HS-(CH₂)ₙ-COOR'
dans laquelle n est tel que défini ci-dessus et R' représente un radical alkyle contenant de 1 à 4 atomes de carbone, avec l'acide maléique, l'acide citraconique ou leur anhydride.

2. Procédé selon la revendication 1, dans lequel on effectue la réaction à une température comprise entre 80 et 140°C, de préférence entre 80 et 120°C, et plus particulièrement au reflux.

3. Procédé selon la revendication 1 ou 2, dans lequel on opère sous une pression allant de 1 à 4 bars, de préférence entre 1 et 2 bars, et plus particulièrement à la pression atmosphérique.

4. Procédé selon l'une des revendications 1 à 3, dans lequel le rapport molaire mercapto-ester/acide ou anhydride maléique ou citraconique est compris entre 0,8 et 1,4, de préférence entre 1 et 1,2, et avantageusement égal à environ 1.

5. Procédé selon l'une des revendications 1 à 4, dans lequel on distille en continu l'alcool R'OH formé.

6. Procédé selon l'une des revendications 1 à 5 pour préparer l'acide carboxyéthylthiosuccinique à partir du mercaptopropionate de méthyle et de l'acide maléique ou son anhydride.
